(19) Europäisches Patentamt
European Patent Office
Office européen des brevets

(11) Veröffentlichungsnummer: **0 084 147**
**B1**

(12) **EUROPÄISCHE PATENTSCHRIFT**

(45) Veröffentlichungstag der Patentschrift: **27.05.87**

(51) Int. Cl.⁴: **C 07 C 109/16**, G 03 G 5/06, G 03 G 5/10

(21) Anmeldenummer: **82111818.9**

(22) Anmeldetag: **20.12.82**

# (54) Neue Phenylhydrazone und deren Verwendung.

(30) Priorität: **16.01.82 DE 3201202**

(43) Veröffentlichungstag der Anmeldung:
**27.07.83 Patentblatt 83/30**

(45) Bekanntmachung des Hinweises auf die Patenterteilung:
**27.05.87 Patentblatt 87/22**

(84) Benannte Vertragsstaaten:
**CH DE FR GB IT LI**

(56) Entgegenhaltungen:
**EP-A-0 001 599**
**DE-A-3 124 396**
**DE-A-3 208 455**
**GB-A-2 094 493**

(73) Patentinhaber: **BASF Aktiengesellschaft**
**Carl-Bosch-Strasse 38**
**D-6700 Ludwigshafen (DE)**

(72) Erfinder: **Neumann, Peter, Dr.**
**Franz-Schubert-Strasse 1**
**D-6908 Wiesloch (DE)**
Erfinder: **Etzbach, Karl-Heinz, Dr.**
**Bensheimer Ring 9A**
**D-6710 Frankenthal (DE)**
Erfinder: **Eilingsfeld, Heinz, Dr.**
**Pierstrasse 9A**
**D-6710 Frankenthal (DE)**
Erfinder: **Hoffmann, Gerhard, Dr.**
**Pappelstrasse 22**
**D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 084 147 B1

**Beschreibung**

Die Erfindung betrifft neue Phenylhydrazone und deren Verwendung.

Die neuen Phenylhydrazone haben die Formel

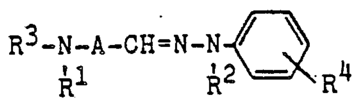 (I),

in der

A für

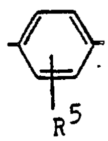 oder 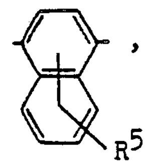 ,

$R^1$ für $C_1$- bis $C_4$-Alkoxyphenyl,

$R^2$ für $C_1$- bis $C_4$-Alkyl oder gegebenenfalls durch $C_1$- bis $C_4$-Alkyl, $C_1$- bis $C_4$-Alkoxy oder Halogen substituiertes Phenyl

$R^3$ für

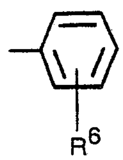 ,

$R^4$, $R^5$ und $R^6$ unabhängig voneinander für Wasserstoff, $C_1$- bis $C_4$-Alkyl, $C_1$- bis $C_4$-Alkoxy oder Halogen stehen, oder

A für

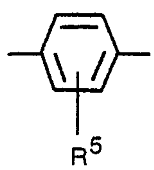 ,

$R^1$ für $C_1$- bis $C_4$-Alkyl, Phen-$C_1$- bis $C_4$-alkyl oder gegebenenfalls durch $C_1$- bis $C_4$-Alkyl, $C_1$- bis $C_4$-Alkoxy oder Halogen substituiertes Phenyl,

$R^3$ für

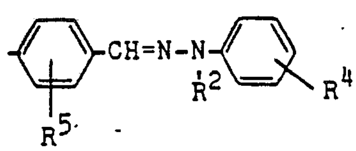 ,

stehen und

$R^2$, $R^4$ und $R^5$ die vorstehend angegebene Bedeutung haben, oder

A für

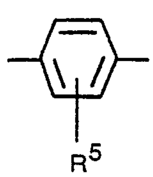 ,

und

$R^1$ und $R^3$ für

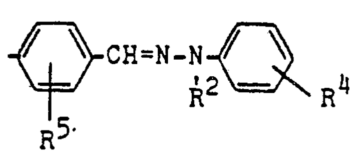 ,

stehen und

$R^2$, $R^4$ und $R^5$ die vorstehend angegebene Bedeutung haben.

Die Phenylhydrazone I sind hervorragend als Ladungsträger transportierende Verbindungen zur Herstellung von elektrophotographischen Aufzeichnungsmaterialien geeignet.

Die mit den Phenylhydrazonen der vorliegenden Erfindung hergestellten elektrophotographischen Aufzeichnungsmaterialien zeigen im Dunkeln eine deutlich geringere Entladung, eine rasche und vollständige Entladung beim Bestrahlen mit aktinischem Licht und eine wesentlich stärkere Differenzierung zwischen unbelichteten und belichteten Stellen, wodurch sich eine deutlich bessere Betonerung der belichteten Aufzeichnungsmaterialien ergibt. In dieser Hinsicht sind die Phenylhydrazone der vorliegenden Erfindung dem aus der EP—B—1599 bekannten 4-Diethylaminobenzaldehyd-N',N'-diphenylhydrazon, das zu den Verbindungen I als nächstliegender Stand der Technik anzusehen ist, deutlich überlegen.

Je nach Substitutionsmuster der Phenylhydrazone I sind für $R^1$ im einzelnen z.B. zu nennen: $C_1$- bis $C_4$-Alkyl: Methyl, Ethyl, n- und i-Propyl, n- und i-Butyl, 2-Butyl; Phen-$C_1$- bis $C_4$-alkyl: Benzyl, 1- oder 2-Phenylethyl, 2- oder 3-Phenylpropyl, 2-, 3- oder 4-Phenylbutyl; gegebenenfalls substituiertes Phenyl: Phenyl, 2-, 3- oder 4-Methylphenyl, 3- oder 4-Ethylphenyl, 3- oder 4-Isopropylphenyl, 4-Butylphenyl, 2-, 3- oder 4-Methoxyphenyl, 4-Ethoxyphenyl, 4-Propoxyphenyl, 4-Butoxyphenyl; 2-, 3-oder 4-Chlorphenyl, 2-, 3- oder 4-Bromphenyl, 4-Jodphenyl und 4-Fluorphenyl.

Halogen bedeutet vorzugsweise Brom und insbesondere Chlor. Vorzugsweise steht $R^1$ für Methoxyphenyl oder Ethoxyphenyl oder für Methyl, Ethyl oder Phenyl.

Für $R^2$ kommen die für $R^1$ vorstehend genannten Alkyl- und gegebenenfalls substituierten Phenylreste in Betracht.

Für $R^2$ sind Methyl, Ethyl und Phenyl bevorzugt.

Für $R^4$, $R^5$ und $R^6$ kommen als $C_1$- bis $C_4$-Alkyl die für $R^1$ genannten in Betracht. Als $C_1$- bis $C_4$-Alkoxy sind im einzelnen Methoxy, Ethoxy, n- und i-Propoxy unbd n- und i-Butoxy zu nennen. Als Halogen kommen Fluor und Jod, vorzugsweise Brom und insbesondere Chlor in Betracht, wobei die Substituenten $R^4$ und $R^6$ in 2-, 3- oder 4-Stellung, vorzugsweise in 3- oder 4-Stellung am Phenylrest stehen.

$R^4$ und $R^5$ stehen vorzugsweise für Wasserstoff.

Für $R^6$ sind Wasserstoff, Methyl, Ethyl, Methoxy und Ethoxy bevorzugt.

Als Reste —A— sind im einzelnen z.B. zu nennen: 1,4-Phenylen, 1,4-Naphthylen, 2- und 3-Methylphenylen-1,4, 2- und 3-Methoxyphenylen-1,4, 2- und 3-Chlor-phenylen-1,4, 2- und 3-Bromphenylen-1,4, 2- und 3-Ethoxyphenylen-1,4; 2- und 7-Methylnaphthylen-1,4 und 2- und 6-Methoxynaphthylen-1,4.

Für A ist 1,4-Phenylen bevorzugt.

Bevorzugt sind wegen ihrer besonders vorteilhaften Eigenschaften Phenylhydrazone der Formel I, in der $R^1$ für Methoxyphenyl oder Ethoxyphenyl, $R^2$ für Methyl, Ethyl oder Phenyl, $R^4$ für Wasserstoff und $R^6$ für Wasserstoff, Methyl, Ethyl, Methoxy oder Ethoxy stehen. Von diesen Hydrazonen sind solche hervorzuheben, in denen A für 1,4-Phenylen steht.

Besonders bevorzugt sind Hydrazone der Formel Ia

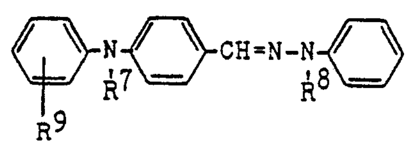

(Ia)

in der $R^7$ für Methoxyphenyl, $R^8$ für Methyl oder Phenyl und $R^9$ für Wasserstoff, Methyl oder Methoxy stehen.

Besonders bevorzugt sind aus anwendungstechnischen Gründen weiterhin Phenylhydrazone der Formel II

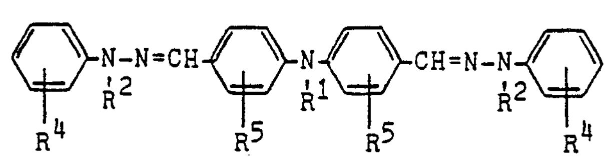

(II),

in der $R^1$ $C_1$- bis $C_4$-Alkyl, Phen-$C_1$- bis $C_4$-alkyl oder gegebenenfalls durch $C_1$- bis $C_4$-Alkyl, $C_1$- bis $C_4$-Alkoxy oder Halogen substituiertes Phenyl oder für einen Rest der Formel

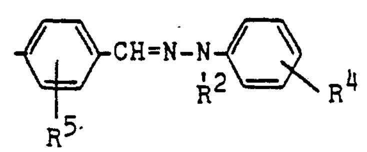

,

$R^2$ für $C_1$- bis $C_4$-Alkyl oder für gegebenenfalls durch $C_1$- bis $C_4$-Alkyl, $C_1$- bis $C_4$-Alkoxy oder Halogen substituiertes Phenyl und $R^4$ und $R^5$ unabhängig voneinander für Wasserstoff, $C_1$- bis $C_4$-Alkyl, $C_1$- bis $C_4$-Alkoxy oder für Halogen stehen.

Von den Hydrazonen der Formel II sind solche hervorzuheben, bei denen $R^1$ und $R^2$ unabhängig voneinander für Ethyl, insbesondere für Methyl oder Phenyl stehen.

Von diesen sind wiederum solche der Formel III besonders bevorzugt:

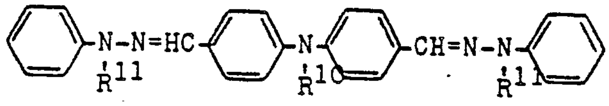

(III)

$R^{11}$ = $-CH_3-$ oder 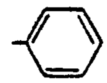

$R^{10}$ = $-CH_3$, 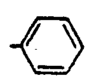 oder 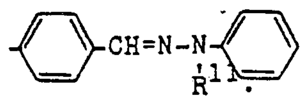.

Als Phenylhydrazone I sind im einzelnen als ganz besonders bevorzugt hervorzuheben:

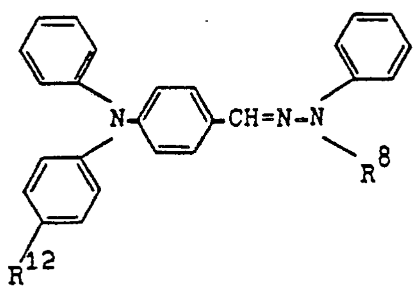

(V)

$R^8$ = $-CH_3$ oder 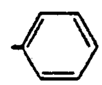

$R^{12}$ = $-OCH_3$

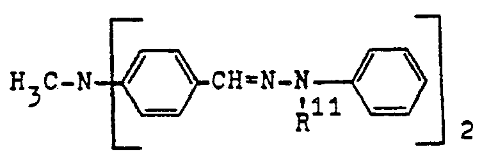

(VI)

$R^{11}$ = $-CH_3$ oder 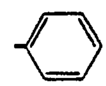

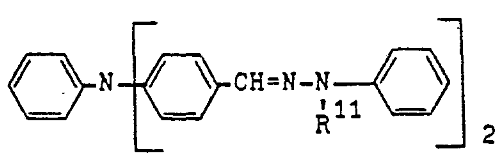

(VII)

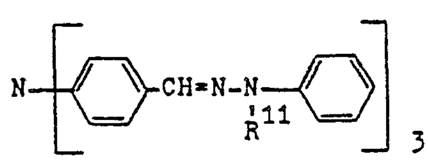

(VIII)

Die Phenylhydrazone I können nach an sich bekannten Verfarhen durch Umsetzen der entsprechenden 4-Aminobenzaldehyde mit den entsprechenden Phenylhydrazinen hergestellt werden.

Vorzugsweise erfolgt die Umsetzung in organischen Lösungsmitteln, gegebenenfalls in Gegenwart einer Säure oder Base. Die Umsetzung erfolgt bei Temperaturen zwischen Raumtemperatur (20°C) und 200°C, vorzugsweise zwischen 50 und 100°C.

Das Umsetzungsprodukt wird für den Fall, daß dieses in dem verwendeten Lösungsmittel löslich ist, durch Zegeben einer nichtlöslichen Flüssigkeit, z.B. Wasser gefällt und durch Abfiltrieren isoliert.

Gegebenenfalls wird das Hydrazon durch Umkristallisieren oder andere übliche Reinigungsoperationen noch weiter gereinigt.

Die als Ausgangsprodukte benötigten Aminobenzaldehyde und Phenylhydrazine sind bekannt oder können nach bekannten Verfahren hergestellt werden.

Die neuen Phenylhydrazone I können in ein- und mehrlagigen elektrophotographischen Aufzeichnungsmaterialien verwendet werden. Im ersteren Fall ist auf einem leitfähigen Träger-material eine Schicht aufgebracht, die die Phenylhydrazone I als Ladungsträger transportierende Substanz und einen bei aktinischer Bestrahlung Ladungsträger erzeugenden Farbstoff und Bindemittel enthält.

Bei mehrlagigen Systemen befindet sich auf dem lietfähigen Trägermaterial eine erste Schicht, in der Ladungsträger durch aktinische Bestrahlung erzeugt werden, und darüber eine Ladungstransportschicht, die I enthält.

Die Herstellung solcher Aufzeichnungsmaterialien und die hierzu benötigten Materialien wie Trägermaterial, Bindemittel und die Ladungsträger erzeugenden Farbstoffe sind bekannt.

Als Trägermaterialien kommen je nach dem Anwendungsgebiet der Aufzeichnungsmaterialien, z.B. Aluminiumbelche, Aluminiumfolien, mit Aluminium bedampfte Polyesterfolien, oder offsettypische vorbehandelte Aluminiumträger in Betracht.

Je nach dem Anwendungsgebiet kommen unterschiedliche Bindemittel in Betracht. So werden für den Kopiersektor z.B. Polyesterharze, PVC, Polystyrol, Polycarbonat verwendet. Für die Anwendung für den Flachdruck sind vor allem alkalilösliche Bindemittel erforderlich. Unter alkalilöslichen Bindemitteln werden solche verstanden, die in wäßrigen oder alkoholischen alkalischen Lösungsmittelsystemen löslich sind. Diese Bindemittel sind hochmolekulare Substanzen, die in Alkali lösliche saure Gruppen wie Anhydrid-, Carboxyl-, phenolische Hydroxyl-, Sulfonsäure-, Sulfonamid- oder Sulfonimidgruppen tragen. Bevorzugt sind Bindemittel mit hohen Säurezahlen, da diese Mittel in alkalisch-wäßrig-alkoholischen Lösungsmittelsystemen besonders leicht löslich sind. Besonders geeignet sind Copolymerisate aus Styrol und Maleinsäureanhydrid, aus Styrol, (Meth)Acrylsäure und (Meth)Acrylsäureestern oder solche aus (Meth)Acrylsäure und (Meth)Acrylsäureestern.

Besonders gut geeignet sind Copolymerisate aus Styrol, Acrylsäure und gegebenenfalls Maleinsäureanhydrid, da in solchen Copolymeren die Säurezahl des Bindemittels besonders leicht an die Erfordernisse angepaßt werden kann. Von diesen Copolymeren haben sich solche aus 55 bis 77 Gew.% Styrol, 5 bis 25 Gew.% Acrylsäure und 0 bis 20 Gew.% Maleinsäureanhydrid als besonders vorteilhaft erwiesen.

Als Ladungsträger erzeugende Stoffe haben sich in einlagigen Systemen z.B. Farbstoffe aus der Reihe der Triarylmethanfarbstoffe, der Xanthen- und/oder Cyaninreihe bewährt. Mit den neuen Hydrazonen I werden besonders gute Ergebnisse mit Malachitgrün (C.I. Basic Green 4; C.I. Nr. 42 000); C.I—. Basic Violet 1, C.I. Nr. 42 535; Kristallviolett; C.H. Basic Violet 10, C.I., Nr. 45 170 oder C.I. Basic Red 1, C.I. 45 160 erzielt.

In mehrlagigen Systemen liegt der Farbstoff oder das Pigment in einer separaten Ladungsträger erzeugenden Schicht vor. Für diesen Fall sind Azofarbstoffe, Phthalocyanine, Isoindolinfarbstoffe und Perylentetracarbonsäurederivate als besonders wirksam bekannt. Hervorragende Ergebnisse werden mit Perylen-3,4:9,10-tetracarbonsäurediimidderivaten erzielt, die in den DE—A 31 10 954 und 31 10 960 genannt werden.

Die folgenden Beispiele sollen die Erfindung zusätzlich erläutern. Die genannten Teile und Prozentangaben beziehen sich auf das Gewicht.

Beispiel 1

6,02 g 4-[N-Phenyl-N-(4-methoxyphenyl)]aminobenzaldehyd und 4,41 g N,N-Diphenylhydrazinhydrochloric wurden in 40 g N-Methylpyrrolidon 3 h bei 60°C gerührt. Nach dem Abkühlen wurden zum Reaktionsgemisch 100 g Wasser getropft. Die Fällung wurde abgesaugt, mit Wasser gewaschen und mit wenig heißen Ethanol digeriert. Nach dem Abkühlen wurde die Fällung abgesaugt, mit wenig Ethanol gewaschen und aus n-Propanol umkristallisiert. Ausbeute: 4,2 g Hydrazon der Formel

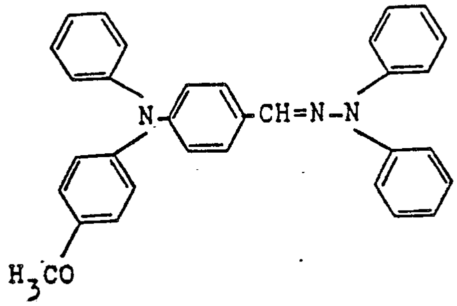

Schmp. 158 bis 160°C

| Analyse $C_{22}H_{27}N_3O$ (M. 470) | C | H | N |
|---|---|---|---|
| ber. | 81.85 | 5,80 | 8.95% |
| gef. | 81,7 | 5,8 | 8,8 |

Beispiel 2

6,02 g 4-[N-Phenyl-N-(4-methoxyphenyl)]aminobenzaldehyd und 2,5-g N-Methyl-N-phenylhydrazin wurden in 40 g NMP 2 h bei 60°C gerührt. Nach dem Abkühlen wurde das Reaktionsgemisch auf 200 g Eiswasser ausgetragen und das sich ölig abscheidende Reaktionsprodukt mit 100 g Toluol aufgenommen. Der nach dem Einengen der Toluolphase erhaltene Rückstand wurde aus Ethanol umkristallisiert. Ausbeute: 3,7 g Hydrazon der Formel

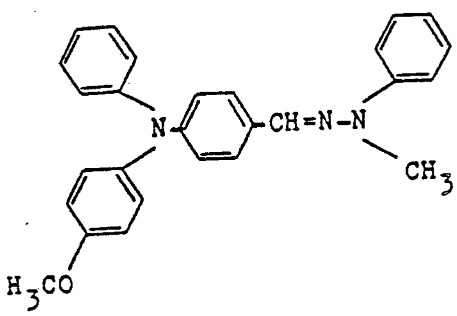

Schmp. 170—172°C

| Analyse: $C_{27}H_{25}N_3O$ (M 408) | C | H | N |
|---|---|---|---|
| ber. | 79,58 | 6,18 | 10,31% |
| gef. | 79,7 | 6,2 | 10,0 % |

Beispiel 3

Eine Mischung aus 4,7 g N-Methyldiphenylamin-4,4'-dicarbaldehyd, 5,0 g N-Methyl-N-phenylhydrazin und 40 g NMP wurde 2 h bei 60°C gerührt. Anschließend wurden 40 g Ethanol zugetropft und unter Rühren auf Raumtemperatur abkühlen gelassen. Der Niederschlag wurde abgesaugt, mit Ethanol und Wasser gewaschen und getrocknet, Nach Umkristallisation aus Ethylenglykolmonoethylether wurden 4,5 g des Hydrazon der Formel

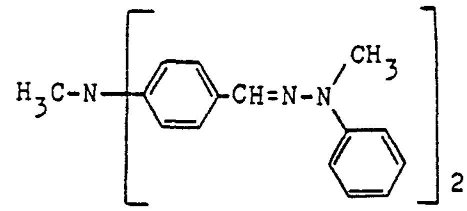

erhalten.

Schmp. 200 bis 202°C

| Analyse: $C_{29}H_{29}N_5$ (M 448) | C | H | N |
|---|---|---|---|
| ber. | 77,82 | 6,53 | 15,65% |
| gef. | 77,6 | 6,4 | 15,7 % |

Beispiel 4

Eine Mischung aus 4,7 g N-Methyldiphenylamin-4,4'-dicarbaldehyd, 8,82 g N,N-Diphenylhydrazinhydrochlorid und 40 g NMP wurde 2 h bei 60°C gerührt. Nach dem Abkühlen auf Raumtemperatur wurde das Reaktionsgemisch auf 100 g Eis ausgetragen, der Niederschlag abgesaugt, mit Wasser gut ausgewaschen und auf Ton getrocknet. Nach Umkristallisation aus Ethanol wurden 2 g des Hydrazone der Formel

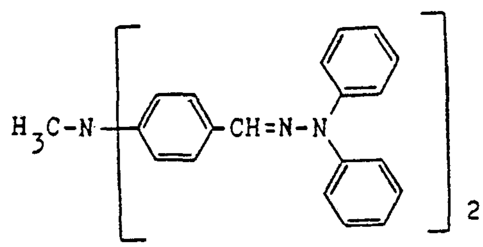

erhalten.

Schmp. 158 bis 159°C

| Analyse: $C_{39}H_{33}N_5$ (M 572) | C | H | N |
|---|---|---|---|
| ber. | 81,93 | 5,82 | 12,25% |
| gef. | 81,7 | 5,8 | 12,1 % |

Beispiel 5

6,0 g eines rohen Gemisches auf Triphenylamin-4-carbaldehyd, Triphenylamin-4,4'-dicarbaldehyd und Triphenylamin-4,4',4''-tricarbaldehyd (erhalten durch Vilsmeier-Formylierung von Triphenylamin) wurden mit 5,0 g N-Methyl-N-phenylhydrazin in 30 g NMP 2 h bei 50 bis 60°C gerührt. Anschließend wurde die Reaktionsmischung auf 200 g Eis ausgetragen. Die Fällung wurde abgesaugt, mit Wasser gut gewaschen und auf Ton getrocknet. Das getrocknete Rohprodukt wurde in n-Propanol suspendiert und kurz zum Sieden erhitzt. Der nach dem Erkalten abgeschiedene Niederschlag wurde abgesaugt und getrocknet. Ausbeute: 5,1 g eines Hydrazongemisches, das als Hauptkomponente das Hydrazon der Formel

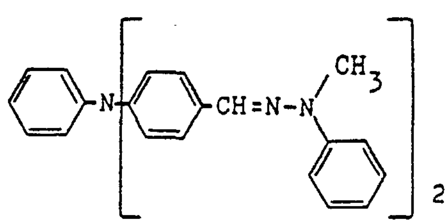

enthält. Schmp. 160 bis 162°C.

Beispiel 6

6,0 g eines rohen, durch Vilsmeier-Formylierung von Triphenylamin erhaltenen Gemisches aus Triphenylamin-4-carbaldehyd, Triphenylamin-4,4'-dicarbaldehyd und Triphenylamin-4,4',4''-tricarbaldehyd wurden mit 8,82 g N,N-Diphenylhydrazinhydrochlorid in 30 g NMP 2 h auf 50 bis 60°C erwärmt. Anschließend wurde das Reaktionsgemisch auf 200 g 50% iges Ethanol ausgetragen. Die Fällung wurde abgesaugt und gut mit Wasser ausgewaschen. Nach dem Trocknen auf Ton das Rohprodukt aus n-Propanol umkristallisiert, wobei man dem Filtrat einige Tropfen Ammoniaklösung zusetzt. Man erhält 1,1 g eines Hydrazongemisches, das als Hauptkomponente das Hydrazons der Formel

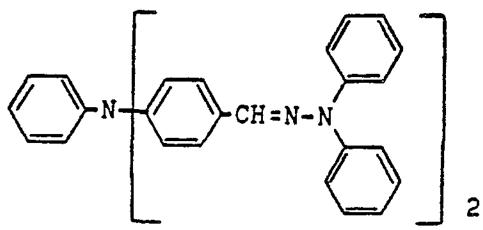

enthält. Schmp. 127 bis 129°C.

Anwendungsbeispiel I

Einlagiges Aufzeichnungsmaterial

55 Teile eines Copolymerisats aus Styrol, Ethylacrylat und Acrylsäure (50:40:10%); K-Wert = 20,5 (2% ige Lösung in DMF)), je 45 Teile eines der in der Tabelle Ia angegebenen Phenylhydrazone I.1 bis I.3 und 0,3 Teile C.I. Basic Violet 1, C.I. Nr. 42535, wurden in 2200 Teilen Essigsäureethylester gelöst und die Lösung auf eine elektrisch leitfähige Unterlage aus Aluminium aufgebracht, daß nach dem Trocknen eine Trockenschichtdicke von 5 µm vorhanden war. Nach dem Ablüften des Lösungsmittels wurde die Schicht 30 min bei 85°C getrocknet.

TABELLE Ia

Verwendete Phenylhydrazone

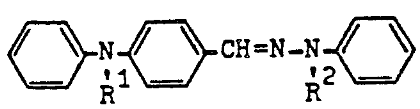

| Beispiel | $R^1$ | $R^2$ | hergestellt nach Beispiel |
|---|---|---|---|
| I.1 | 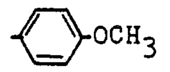 | 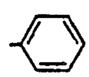 | 1 |
| I.2 | 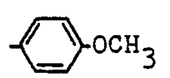 | $-CH_3$ | 2 |
| I.3 | 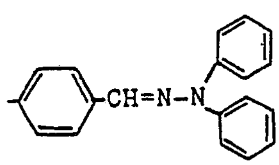 | 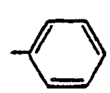 | 6 |
| Vgl. | 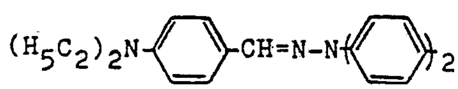 | | EP—B 1599 |

Die Schichten wurden dann mit einer Gleichspannungscorona von −8,5 kV in 1 cm Abstand gleichmäßig beladen und dann mit weißem Licht einer Beleuchtungsstärke von ungefähr 0,85 $mW.cm^{-2}$ belichtet. An den so behandelten Platten wurden gemessen:

Meßgröße A: nach einer Beladungszeit von 20 Sekunden erreichtes Oberflächenpotential in Volt;
Meßgröße B Potentialabfall in %, bezogen auf Meßgröße A, im Dunkeln innerhalb von 20 Sekunden;
Meßgröße C: durch die aktinische Belichtung hervorgerufener Potentialabfall in %, bezogen auf das Ausgangspotential unmittelbar vor der Belichtung;
Meßgröße D: Potentialänderung pro Sekunde zu Beginn der Belichtung;
Meßgröße E: Zeit, innerhalb der das vor der Belichtung vorhandene Potential bei aktinischer Belichtung auf die Hälfte abgefallen ist;
Meßgröße F: Potentialdifferenz zwischen belichteten und unbelichteten Flächen der beladenen Schichten.

Die Meßergebnisse sind in der Tabelle Ib) zusammengestellt.

TABELLE Ib

| | Meßgröße | | | | | |
|---|---|---|---|---|---|---|
| Beispiel | A [V] | B [%] | C [%] | D [V/s] | E [ms] | F [V] |
| I.1 | 1070 | 36 | 99 | 6700 | 140 | 680 |
| I.2 | 700 | 40 | 97 | 4500 | 190 | 400 |
| I.3 | 850 | 38 | 99 | 4900 | 140 | 520 |
| Vgl. | 400 | 48 | 83 | 3450 | 305 | 210 |

Tabelle Ib) zeigt, daß Schichten, welche die erfindungsgemäßen Phenylhydrazone I enthalten, eine geringere Entladung im Dunkeln, eine raschere und vollständigere Entladung bei aktinischer Belichtung zeigen und hauptsächlich eine wesentlich stärkere Differenzierung zwischen belichteten und unbelichteten Stellen aufweisen. Die zuletzt erwähnte Eigenschaft ergibt eine wesentlich bessere Betonerung der bildmäßig belichteten Aufzeichnungsmaterialien.

Anwendungsbeispiel II

Einlagiges Aufzeichnungsmaterial

60 Teile eines Copolymerisates aus Styrol und Methacrylsäure (76:24%; K-Wert 39,8 (2% ige Lösung in DMF)), je 40 Teile der in Tabelle IIa angegebenen Phenylhydrazone II.1 bis II.4 und 0,6 Teile C.I. Basic Violet 10; C.I. Nr. 45 170 wurden im Tetrahydrofuran gelöst. Die Homogene Lösung wird mit einer Reverse-Coating Anlage in einer solchen Naßschichtdicke auf eine fein gebürstete Aluminiumoberfläche aufgebracht, daß nach dem Trocknen eine Trockenschicht von 3,5 ± 0,2 µm Dicke vorliegt.

TABELLE IIa

Verwendete Phenylhydrazone

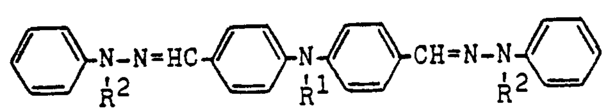

| Anwendungsbeispiel | $R^1$ | $R^2$ | |
|---|---|---|---|
| II.1 | 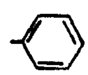 | 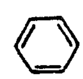 | |
| II.2 | 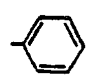 | $-CH_3$ | |
| II.3 | $-CH_3$ | 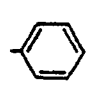 | |
| II.4 | $-CH_3$ | $-CH_3$ | |
| Vergl. | 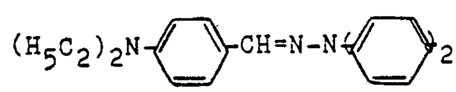 | | EP—B 1599 |

Die Schichten wurden wie im Anwendungsbeispiel I angegeben behandelt und den behandelten Platten die Meßgrößen A bis F bestimmt. Die Meßergebnisse sind in der folgenden Tabelle IIb) zusammengestellt.

TABELLE IIb

| | Meßgröße | | | | | |
|---|---|---|---|---|---|---|
| Beispiel | A [V] | B [%] | C [%] | D [V/s] | E [ms] | F [V] |
| II.1 | 1100 | 41 | 99 | 5300 | 175 | 640 |
| II.2 | 1000 | 35 | 99 | 5550 | 175 | 640 |
| II.3 | 1250 | 38 | 99 | 3600 | 220 | 765 |
| II.4 | 1580 | 39 | 99 | 4600 | 200 | 950 |
| Vgl. | 400 | 48 | 83 | 3450 | 305 | 210 |

Tabelle IIb zeigt, daß Schichten, welche die erfindungsgemäßen Phenylhydrazone I enthalten, eine geringere Entladung im Dunkeln, eine raschere und vollständigere Entladung bei aktinischer Belichtung zeigen und hauptsächlich eine wesentlich stärkere Differenzierung zwischen belichteten und unbelichteten Stellen aufweisen. Die zuletzt erwähnte Eigenschaft ergibt eine wesentlich bessere Betonerung der bildmäßig belichteten Aufzeichnungsmaterialien.

**Patentansprüche**

1. Phenylhydrazone der allgemeinen Formel

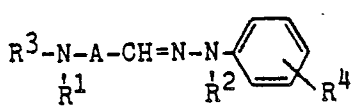

(I),

in der

A für

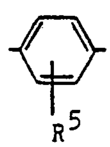

oder

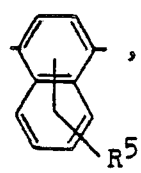

,

$R^1$ für $C_1$- bis $C_4$-Alkoxyphenyl,

$R^2$ für $C_1$- bis $C_4$-Alkyl oder gegebenenfalls durch $C_1$- bis $C_4$-Alkyl, $C_1$- bis $C_4$-Alkoxy oder Halogen substituiertes Phenyl

$R^3$ für

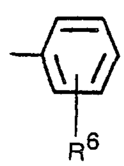

,

$R^4$, $R_2$ und $R^6$ unabhängig voneinander für Wasserstoff, $C_1$- bis $C_4$-Alkyl, $C_1$- bis $C_4$-Alkoxy oder Halogen stehen, oder

A für

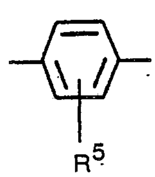

,

$R^1$ für $C_1$- bis $C_4$-Alkyl, Phen-$C_1$- bis $C_4$-alkyl oder gegebenenfalls durch $C_1$- bis $C_4$-Alkyl, $C_1$- bis $C_4$-Alkoxy oder Halogen substituiertes Phenyl,

$R^3$ für

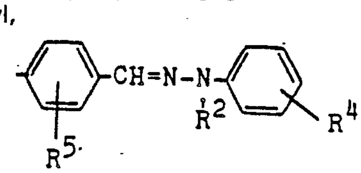

,

stehen und

$R^2$, $R^4$ und $R^5$ die vorstehend angegebene Bedeutung haben, oder

A für

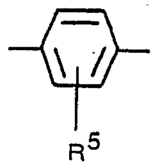,

und

$R^1$ und $R^3$ für

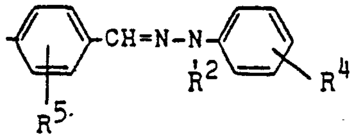,

stehen und

$R^2$, $R^4$ und $R^5$ die vorstehend angegebene Bedeutung haben.

2. Phenylhydrazone gemäß Anspruch 1, dadurch gekennzeichnet, daß in der Formel A für 1,4-Phenylen steht.

3. Phenylhydrazone gemäß Anspruch 1 oder 2, dadurch gekennzeichnet, daß in der Formel $R^1$ für Methoxyphenyl oder Ethoxyphenyl, $R^2$ für Methyl, Ethyl oder Phenyl, $R^4$ für Wasserstoff und $R^6$ für Wasserstoff, Methyl, Ethyl, Methoxy oder Ethoxy stehen.

4. Phenylhydrazone gemäß Anspruch 1, gekennzeichnet durch die Formel

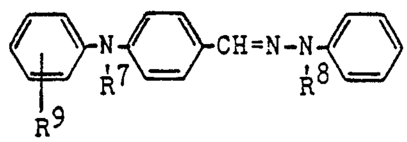

(Ia)

in der

$R^7$ für Methoxyphenyl,

$R^8$ für Methyl oder Phenyl und

$R^9$ für Wasserstoff, Methyl oder Methoxy stehen.

5. Phenylhydrazone gemäß Anspruch 1, gekennzeichnet durch die Formel

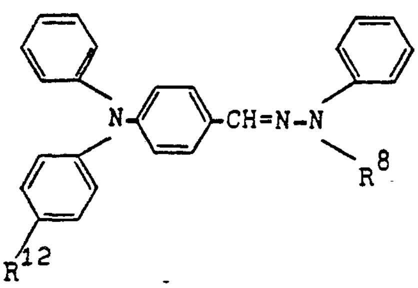

(V)

in der $R^8$ für Methyl oder Phenyl und $R^{12}$ für Methoxy stehen.

6. Phenylhydrazone gemäß Anspruch 1, gekennzeichnet durch die Formel

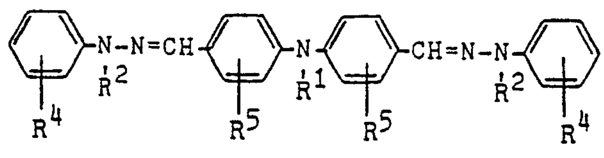

(II),

in der

$R^1$ für $C_1$- bis $C_4$-Alkyl, Phen-$C_1$- bis $C_4$-Alkyl, oder für gegebenenfalls durch $C_1$- bis $C_4$-Alkyl, $C_1$- bis $C_4$-Alkoxy oder Halogen substituiertes Phenyl oder für

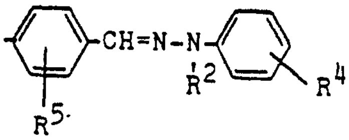,

stehen und

$R^2$, $R^4$ und $R^5$ die in Anspruch 1 angegebene Bedeutung haben.

7. Phenylhydrazone gemäß Anspruch 1, 2, 3 oder 6, dadurch gekennzeichnet, daß $R^5$ Wasserstoff ist.

8. Phenylhydrazon gemäß Anspruch 6, dadurch gekennzeichnet, daß $R^2$ für Methyl, Ethyl oder Phenyl und $R^5$ für Wasserstoff stehen.

9. Phenylhydrazone gemäß Anspruch 6, 7 oder 8, dadurch gekennzeichnet, daß $R^1$ für Methyl, Ethyl oder Phenyl steht.

10. Phenylhydrazone gemäß Anspruch 6 oder 8, dadurch gekennzeichnet, daß $R^4$ und $R^5$ Wasserstoff sind.

11. Phenylhydrazone gemäß Anspruch 6, dadurch gekennzeichnet, daß $R^4$ und $R^5$ für Wasserstoff und $R^1$ und $R^2$ unabhängig voneinander für Methyl oder Phenyl stehen.

12. Verwendung der Hydrazone gemäß den Ansprüchen 1 bis 11 als Ladungsträger transportierende Verbindungen in elektrophotographischem Aufzeichnungsmaterial.

**Revendications**

1. Phénylhydrazones de formule générale

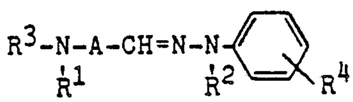

(I),

dans laquelle

A est mis pour

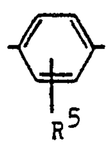

ou

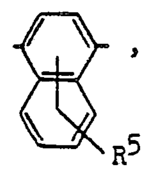

,

$R^1$ pour alcoxyphényle en $C_1$ à $C_4$,

$R^2$ pour alkyle en $C_1$—$C_4$-ou phényle éventuellement substitué par alkyle en $C_1$—$C_4$, alcoxy en $C_1$- à $C_4$- ou halogène,

$R^3$ pour

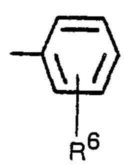

,

$R^4$, $R^5$ et $R^6$, indépendamment l'un de l'autre, pour hydrogène, alkyle en $C_1$ à $C_4$, alcoxy en $C_1$ à $C_4$ ou halogène ou

A pour

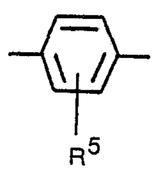

,

$R^1$ pour alkyle en $C_1$ à $C_4$, phen-alkyle (en $C_1$—$C_4$) ou phényle éventuellement substitué par alkyle en $C_1$ à $C_4$, alcoxy en $C_1$ à $C_4$ ou halogène,

$R^3$ pour

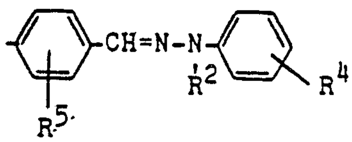

,

et

$R^2$, $R^4$ et $R^5$ ont les significations sus-indiquées, ou

A est mis pour

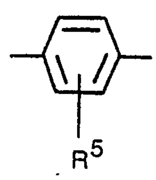

, et

$R^1$ et $R^3$ pour 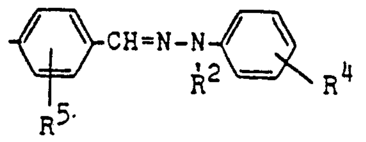 ,

et

$R^2$, $R^4$ et $R^5$ ont les significations sus-indiquées.

2. Phénylhydrazones selon la revendication 1, caractérisés par le fait que, dans la formule, A est mis pour phénylène-1,4.

3. Phénylhydrazones selon la revendication 1 ou 2, caractérisées par le fait que, dans la formule, $R^1$ est mis pour méthoxyphényle ou éthoxyphényle, $R^2$ pour méthyle, éthyle ou phényle, $R^4$ pour hydrogène et $R^6$ pour hydrogène, méthyle, méthoxy ou éthoxy.

4. Phénylhydrazones selon la revendication 1, caractérisées par la formule

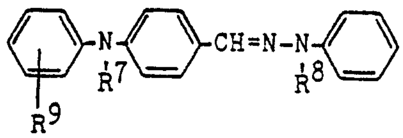

(Ia)

dans laquelle

$R^7$ est mis pour méthoxyphényle,

$R^8$ pour méthyle ou phényle et

$R^9$ pour hydrogène, méthyle ou méthoxy.

5. Phénylhydrazones selon la revendication 1, caractérisées par la formule

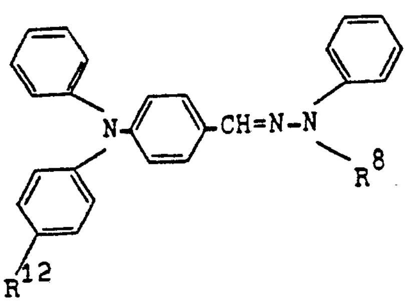

(V)

dans laquelle $R^8$ est mis pour méthyle ou phényle et $R^{12}$ pour méthoxy.

6. Phénylhydrazones selon la revendication 1, caractérisées par la formule

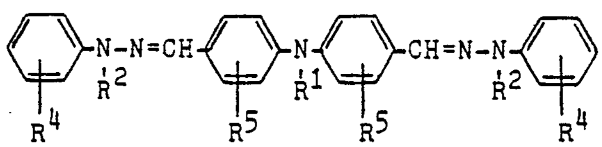

(II),

dans laquelle

$R^1$ est mis pour alkyle en $C_1$ à $C_4$, phen- alkyle en $C_1$ à $C_4$ ou phényle éventuellement substitué par alkyle en $C_1$ à $C_4$, alcoxy en $C_1$—$C_4$ ou halogène ou pour

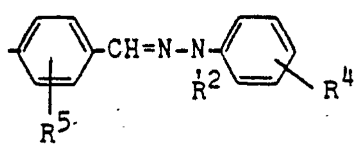 ,

et

$R^2$, $R^4$ et $R^5$ ont les significations données dans la revendication 1.

7. Phénylhydrazones selon les revendications 1, 2, 3 ou 6, caractérisées par le fait que $R^5$ est l'hydrogéne.

8. Phénylhydrazones selon la revendication 6, caractérisées par le fait que $R^2$ est mis pour méthyle, éthyle ou phényle et $R^5$ pour hydrogène.

9. Phénylhydrazones selon la revendication 6, 7 ou 8, caractérisées par le fait que $R^1$ est mis pour méthyle, éthyle ou phényle.

10. Phénylhydrazones selon la revendication 6 ou 8, caractérisées par le fait que $R^4$ et $R^5$ sont de l'hydrogène.

11. Phénylhydrazones selon la revendication 6, caractérisées par le fait que $R^4$ et $R^5$ sont mis pour hydrogène et $R^1$ et $R^2$, indépendant l'un de l'autre, pour méthyle ou phényle.

12. Utilisation des hydrazones selon les revendications 1 à 11, comme composés transportant des porteurs de charges dans des matériaux d'enregistrement électrophotographiques.

**Claims**

1. A phenylhydrazone of the general formula

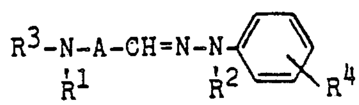 (I),

where

A is

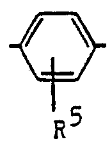 or 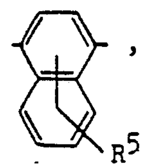 ,

$R^1$ is $C_1$—$C_4$-alkoxyphenyl,

$R^2$ is $C_1$—$C_4$-alkyl or is phenyl which is unsubstituted or substituted by $C_1$—$C_4$-alkyl, $C_1$—$C_4$-alkoxy or halogen.

$R^3$ is

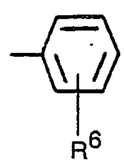 ,

and

$R^4$, $R^5$ and $R^6$ independently of one another are hydrogen, $C_1$—$C_4$-alkyl, $C_1$—$C_4$-alkoxy or halogen, or

A is

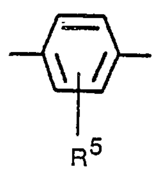 ,

$R^1$ is $C_1$—$C_4$-alkyl or phenyl-$C_1$—$C_4$-alkyl, or is phenyl which is unsubstituted or substituted by $C_1$—$C_4$-alkyl, $C_1$—$C_4$-alkoxy or halogen,

$R^3$ is

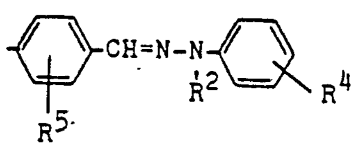 ,

and

$R^2$, $R^4$ and $R^5$ have the abovementioned meanings, or

A is

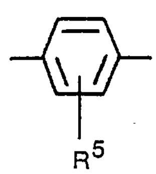 ,

$R^1$ and $R^3$ are each

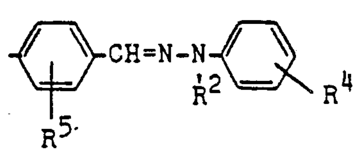 ,

and

$R^2$, $R^4$ and $R^5$ have the abovementioned meanings.

2. A phenylhydrazone as claimed in claim 1, wherein, in the formula, A denotes 1,4-phenylene.

3. A phenylhydrazone as claimed in claim 1 or 2, wherein, in the formula,

$R^1$ is methoxyphenyl or ethoxyphenyl, $R^2$ is methyl, ethyl or phenyl,

$R^4$ is hydrogen, and $R^6$ is hydrogen, methyl, ethyl, methoxy or ethoxy.

4. A phenylhydrazone as claimed in claim 1, of the formula

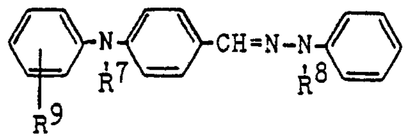

(Ia)

where

$R^7$ is methoxyphenyl,

$R^8$ is methyl or phenyl, and

$R^9$ is hydrogen, methyl or methoxy.

5. A phenylhydrazone as claimed in claim 1, of the formula

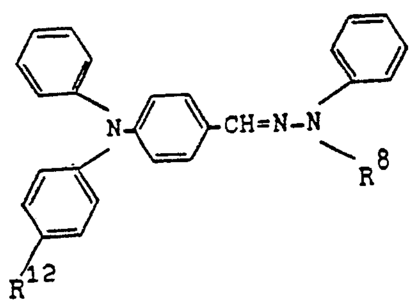

(V)

where $R^8$ is methyl or phenyl, and $R^{12}$ is methoxy.

6. A phenylhydrazone as claimed in claim 1, of the formula

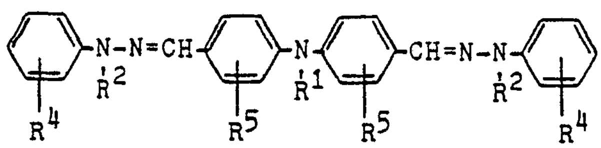

(II),

where

$R^1$ is $C_1$—$C_4$-alkyl or phenyl-$C_1$—$C_4$-alkyl, or is phenyl which is unsubstituted or substituted by $C_1$—$C_4$-alkyl, $C_1$—$C_4$-alkoxy or halogen, or is a radical of the formula

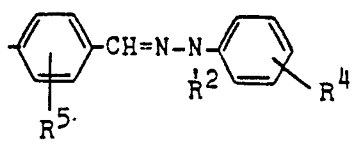

,

and

$R^2$, $R^4$ and $R^5$ have the meanings given in claim 1.

7. A phenylhydrazone as claimed in claim 1, 2, 3 or 6, wherein $R^5$ is hydrogen.

8. A phenylhydrazone as claimed in claim 6, wherein $R^2$ is methyl, ethyl or phenyl, and $R^5$ is hydrogen.

9. A phenylhydrazone as claimed in claim 6, 7 or 8, wherein $R^1$ is methyl, ethyl or phenyl.

10. A phenylhydrazone as claimed in claim 6 or 8, wherein $R^4$ and $R^5$ are each hydrogen.

11. A phenylhydrazone as claimed in claim 6, wherein $R^4$ and $R^5$ are each hydrogen, and $R^1$ and $R^2$ independently of one another are methyl or phenyl.

12. The use of a hydrazone as claimed in claim 1 to 11 as charge-carrier-transporting compound in electrophotographic recording material.